# EUROPEAN PATENT SPECIFICATION

(11) **EP 4 306 208 B1**
(45) Date of publication and mention of the grant of the patent: **04.11.2026**
(21) Application number: 23184994.4
(22) Date of filing: 12.07.2023
(51) Int. Cl.: B01J 13/04, A61K 51/12, B01J 13/20

(54) **PROCESS FOR MAKING ACTIVE CARRIERS**
VERFAHREN ZUR HERSTELLUNG AKTIVER TRÄGER
PROCÉDÉ DE FABRICATION DE PORTEURS ACTIFS

(30) Priority: 13.07.2022 IT 202200014707
(43) Date of publication of application: 17.01.2024
(73) Proprietor: Cester, Achille, 20090 Opera (MI) (IT); Chauvie, Stephane, 12010 Vignolo (CN) (IT); Grosso, Maurizio, 12100 Cuneo (CN) (IT); Cester, Lucrezia, London (GB); Verificatori Associati Italiani S.r.l., 27055 Rivanazzano Terme (PV) (IT)
(72) Inventor: CESTER, Achille, 20090 Opera (MI) (IT); CHAUVIE, Stephane, 12010 Vignolo (CN) (IT); CESTER, Lucrezia, London (GB)
(74) Representative: Lunati & Mazzoni S.r.L.

(56) References cited:
- WO-A1-2012/057401
- WO-A1-2016/191247
- WO-A2-2009/140266
- US-A1- 2015 320 895
- JUNG S H ET AL: "Preparation of radioactive core-shell type ^1^9^8Au@SiO"2 nanoparticles as a radiotracer for industrial process applications", APPLIED RADIATION AND ISOTOPES, ELSEVIER, OXFORD, GB, vol. 68, no. 6, 1 June 2010 (2010-06-01), pages 1025 - 1029, XP026947028, ISSN: 0969-8043, [retrieved on 20100125], DOI: 10.1016/J.APRADISO.2010.01.021

## Description

This invention relates to a process for making active carriers of the type specified in the preamble of the first claim.

The object of this invention is a process for materials that perform the function of carriers for radionuclides or other non-radioactive molecules and drugs and that are mainly, but not exclusively, applied in the medical field.

Technologies that enable the transport of powdered materials using materials that perform the function of carriers are currently known. In particular, these powdered materials may contain radionuclides or other non-radioactive molecules and drugs. The materials containing radionuclides may also be transported in the form of pellets.

Alternatively, the powders containing radionuclides may be deposited on carriers using suitable deposition techniques.

The carriers on which radionuclides are deposited are typically used in the medical sector, in diagnostic and treatment techniques, but also in the metallurgical industry and, in particular, in analyses aimed at highlighting irregularities in material, detecting the presence of cracks and fractures, via the surface coating.

A specific treatment example is radioembolization, the treatment of solid tumours (e.g., original, and secondary tumours in the liver, prostate cancer, kidney, lung, and brain tumours, etc.).

One critical issue linked to the use of these materials lies in the fact that they must enable efficient transport of radioactive elements inside the patient's body. It is, thus, necessary to ensure that these materials effectively and quickly reach the areas the treatment involves with these materials. This need is linked to the fact that the materials containing radionuclides emit ionising radiation and this radiation must preferentially interact with the organs and tissues to be treated, for example, the site of a tumour, so as to avoid damage to organs or healthy tissues, via the appropriate choice of the radionuclide used and its non-chemical toxicity for the body, once it has decayed. In addition, the carrier must be compatible with the tissue.

To make the administration of radionuclides efficient, the carriers typically have dimensions that may vary from a few µm to a few hundred µm. Therefore, the carriers used in this kind of application are typically nanoparticles. In particular, one of the preferred shapes for increasing the exposed surface area is the sphere. Nanospheres are, thus, the type of carrier that is most efficient for these applications and they are also ideal for administration. In some cases, the radionuclides are fused to the carrier, before being activated; in any case, this methodology entails less carrier efficiency compared to those comprising the deposition on the surface of the carriers.

Both in diagnostic and treatment techniques, these materials enable administration by injection (for example intravenously, intraarterially, percutaneously, or topically), as well as a suitable dose for the use purposes that does not entail risks for the patient.

For the production of these materials, various production techniques were developed. Some techniques, as mentioned above, comprise the fusion of carriers and nuclides, which are then radiated. For these techniques, therefore, the radiation is not selective; thus, the activation also involves the carrier, with the consequent morphological and functional deterioration, including due to radiation heat.

For these reasons, the most widely used techniques are based on the coating of the carrier with materials containing nuclides subsequently subjected to activation.

Typically, these techniques use the deposition of powders via electrostatic deposition on the nanospheres, via deposition using sputtering, or other techniques that involve mechanical means for deposition.

The activation of the nuclides is typically carried out within cyclotrons.

US 2015/320895 A1 discloses a method for making a radioactive nanoparticle comprising a metal nanoparticle core and an outer metal shell comprising a metallic radioisotope by a galvanic replacement reaction.

The prior art that has been developed comprises some major drawbacks.

In particular, in the known techniques, there is poor adhesion of the transported powder to the carrier, with the resulting loss of material, which detaches from the carrier and is not used. In addition to the decrease in activity required for application, this drawback causes an increase in costs, which are already high, as often happens for materials containing radionuclides.

In addition, the loss of material, the result of poor adhesion to the carrier, entails ecological problems and risks to human health.

Finally, the layer of powder deposited on the carrier tends to form aggregates that entail a reduction in activity since the materials containing radionuclides need to be activated before they are used.

In this situation, the technical task behind this invention is to devise a process for making carriers covered by radionuclides able to substantially overcome at least part of the drawbacks mentioned above.

Within the sphere of said technical purpose one important aim of the invention is to obtain carriers able to minimise the losses of powder containing radionuclides due to poor adhesion to the carrier.

Another purpose of the invention is to use neutron activation to generate radionuclides, instead of the conventional activation using cyclotrons.

Another important purpose of the invention is to make carriers that enable the transport of powders containing radionuclides in constant quantities.

Another purpose of the invention is to enable the transport and simpler dosing of the material containing radionuclides.

The technical purpose and specified aims are achieved with a process for making active carriers covered by radionuclides as claimed in the appended claim 1. Preferred technical solutions are highlighted in the dependent claims.

The invention is as defined in the appended claims.

The features and advantages of the invention are clarified below by the detailed description of preferred embodiments of the invention, with reference to the accompanying drawings, wherein:

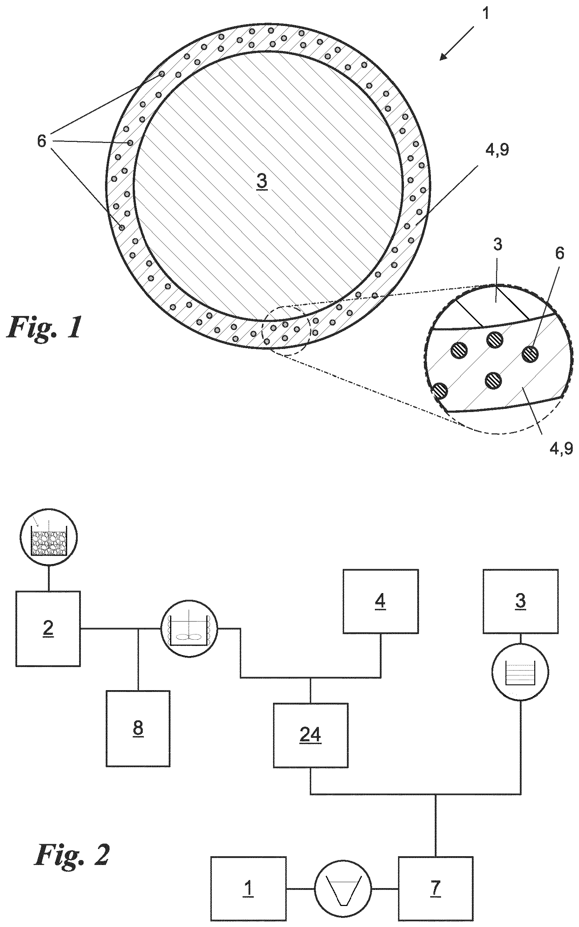

**Fig. 1** shows a schematic view in normal section of an active carrier according to the invention, with a detail of the surface layer;
**Fig. 2** illustrates a diagram of a process for creating said carriers.

In this document, the measures, values, shapes, and geometric references (such as perpendicularity and parallelism), when used with words like "about" or other similar terms such as "approximately" or "substantially", are to be understood as except for measurement errors or inaccuracies due to production and/or manufacturing errors and, above all, except for a slight divergence from the value, measure, shape, or geometric reference which it is associated with. For example, if associated with a value, such terms preferably indicate a divergence of no more than 10% from the value itself.

Furthermore, when terms such as "first", "second", "upper", "lower", "main" and "secondary" are used, they do not necessarily identify an order, relationship priority or relative position, but they can simply be used to distinguish different components more clearly from one another.

Unless otherwise specified, as is apparent from the following discussions, it is considered that terms such as "computer science", "determination", "calculation" or similar, refer to the computer action and/or processes or similar electronic computing devices that manipulate and/or transform data represented as physical, such as electronic quantities of registers of an information system and/or memory, other data similarly represented as physical quantities within computer systems, registers or other devices for storing, transmitting or displaying information.

Unless otherwise stated, the measurements and data reported in this text shall be considered as performed in International Standard Atmosphere ICAO (ISO 2533:1975).

With reference to the drawings, reference numeral **1** globally denotes the active carriers according to the invention.

The subject of the invention comprises a process for making active carriers 1. The active carriers 1 are materials with nanometric dimensions optimised for the transport of species containing radionuclides.

The active carriers 1 typically comprise nanospheres **3** (Nano, in this case, is a commercially used term that has nothing to do with the order of size called thus in the SI; in fact, the term microspheres is also used). Nanospheres 3 comprise inorganic material. Specifically, nanospheres 3 preferably comprise materials like pure silicon, meaning silicon with an impurity level below 0.1%. Nanospheres 3 may also comprise glass without traces of CO₂ and sodium, or with said elements in quantities below 5%.

In addition, nanospheres 3 preferably comprise at least one large inner cavity. The configurations comprising nanospheres 3 including inner cavities have the advantage of saving material and a resulting lightening of the active carriers 1.

The active carriers 1 comprise an organofunctional material **4**, comprising at least epoxy-silane and preferably amino-silane, deposited on the surface of the nanospheres 3. The organofunctional material 4 preferably comprises silanols, able to react with hydroxy groups (-OR) of the chosen metals, which will be discussed below, with the addition of a functional group, such as, for example, the amine group (-NH₂).

The active carriers 1 comprise an activated powder **6** containing radionuclides. The activated powder 6 is obtained starting with a powder **2** subjected to an activation process, consisting in its exposure to neutron beams within a thermal-neutron reactor.

The powder 2 preferably comprises a metal belonging to the lanthanide series.

In particular, the powder 2 preferably comprises holmium oxide. This material is known for being effective for applications in the medical field and, in particular, for treatment purposes.

The organofunctional material 4, in the preferred embodiments, preferably comprises silanols that form siloxane bonds.

In the embodiments that comprise holmium oxide, the average diameter of the particles preferably falls within a range between 0.05 µm and 10 µm, more preferably between 0.07 µm and 7 µm, even more preferably between 0.1 µm and 5 µm.

The choice of the organofunctional material 4 based on the type of powder 2 used is due to the affinity of the powder for the organofunctional material 4 chosen.

In some technical solutions, the organofunctional material 4 comprises at least epoxy-silane and preferably amino-silane. In this type of embodiment, epoxy-silane is used, but the efficacy of the final active carriers 1 is increased when the coating 5 also comprises amino-silane mixed with epoxy-silane using a catalyser that speeds up the reaction between the epoxy-silane and the amino-silane, to obtain a homogeneous layer.

The active carriers 1 comprise a layer **9.** The layer 9 comprises the organofunctional material 4, the activated powder 6 and is deposited on the surface of the nanospheres 3 according to the steps described in the process.

The layer 9 has the function of enabling an effective intermediate bond between the radionuclides and the nanospheres 3, with a resulting improvement in the activity of the radionuclides.

The active carriers 1 are made using a process comprising a preparing step of the nanospheres 3 in inorganic material. In this step, nanospheres are selected that have a diameter that preferably falls within a range between 10 µm and 200 µm, more preferably between 15 µm and 100 µm, more preferably between 20 µm and 60 µm, more preferably between 30 µm and 50 µm, still more preferably between 37 µm and 45 µm. The nanospheres 3 are preferably selected using sieves.

The process comprises a step for crushing the coarse matrix from which the powder 2 is obtained and its grinding. The crushing of the coarse matrix is preferably carried out using a mill. This step is advantageous in terms of the efficiency of the active carriers 1. In fact, the process of grinding the powder 2 reduces the formation of agglomerates that may enter and become part of the coating 5 structure. The formation of agglomerates in the coating 5 ensures that only a part of the powder 2 is converted into activated powder 6, since a part of the powder 2 particles is found inside the agglomerates. Thus, they are screened from the particles above and will expose a smaller area to the neutron beam during the activation step. This effect has repercussions on the final level of active carriers 1 activity, which will be less than what can be achieved when all the powder 2 is converted into activated powder 6.

The grinding step comprises the dispersion of the powder 2 in a dispersing agent **8**, preferably comprising isopropanol or silane gas. The addition of the dispersing agent 8 entails an improvement in the separation of the particles of the powder 2, which has the effect of further increasing the final efficiency of the active carriers 1. At the end of the grinding step, a dispersion of the powder 2 is preferably obtained and the dispersion is poured into a container.

The process comprises a step for preparing the organofunctional material 4. In this step, the organofunctional material 4 chosen is preferably poured into a container.

The process advantageously comprises an initial step of synthesising inactive carriers **7**. The inactive carriers 7 comprise nanospheres 3, the coating 5 deposited on the surface of the nanospheres 3 and the powder 2 bound to the coating 5.

The initial step of synthesis comprises a sub-step of incorporating powder 2 into the organofunctional material 4. The incorporation sub-step enables the bonding of the powder 2 to the polymer material.

The incorporation sub-step preferably comprises the preparation of a dispersion **24** of the powder 2 in the organofunctional material 4. In the variants of the process that comprise the isopropanol dispersing agent 8, in the powder 2 grinding step, to make the dispersion 24, the organofunctional material 4 is poured into the mixture containing the powder 2 dispersed in the dispersing agent 8. In a preferred embodiment, to make the dispersion 24, dipodal polysiloxane is poured into a powder mixture 2 in holmium oxide and dispersed in the isopropanol dispersing agent 8.

The initial step of synthesis comprises a sub-step of depositing the organofunctional material 4 on the surface of the nanospheres 3 to create the coating 5.

In a preferred variant of the process, the dispersion 24, thus comprising the organofunctional material 4, is preferably deposited on the nanospheres 3. More specifically, the deposition preferably occurs via immersion of the nanospheres 3 in the dispersion 24, with subsequent evaporation of the dispersing agent 8. In this way, the inactive carriers 7, wherein the powder 2 is bound to the nanospheres 3, is obtained via the coating 5.

In the solution comprising holmium oxide and dipodal polysiloxane, the ratio between the average diameter of the particles of holmium oxide and the diameter of the nanospheres 3 preferably ranges between 1:10 and 1:500, more preferably between 1:20 and 1:450, still more preferably between 1:20 and 1:150.

Again, with reference to this preferred solution, the percentage ratios between the masses of the coating 5 and the nanospheres 3 preferably ranges between 0.05% and 6%, more preferably between 0.1% and 5%, still more preferably between 0.1% and 4%.

In addition, the percentage ratios between the masses of the holmium oxide and the nanospheres 3 preferably ranges between 0.1% and 20%, more preferably between 0.3 % and 15%, still more preferably between 0.4% and 11 %.

The process comprises a second step for synthesising the active carriers 1 wherein the active carriers 7 are activated by generating radionuclides in the powder 2, and the consequent transformation of the powder 2 into activated powder 6, and, consequently, of the inactive carriers 7 into active carriers 1.

The generation of the radionuclides is carried out by exposing the inactive carriers 7 to a neutron beam to engender the reaction of neutron radiative capture. The neutron radiative capture reaction entails the generation of radionuclide children that emit gamma and/or beta rays.

In particular, the activation process is preferably carried out using a thermal neutron reactor using the neutron radiative capture reaction.

The thermal neutron reactor used in the activation process is preferably the TRIGA (Training Research and Isotopes-production General Atomics) Mark II reactor.

The TRIGA Mark II reactor is a reactor comprising a cooling tank, in part moderated with light water and that uses a fuel composed of 8% uranium (enriched to 20%), 1% hydrogen, and 91% zirconium.

The TRIGA Mark II reactor is designed to process at a maximum power in the stationary state of 250 kW. The reactor core has the shape of a straight cylinder and contains 90 housings arranged in 5 concentric rings inside a circular grill, which also performs the function of sample carrier. The housings contain combustible elements, graphite elements, control bars or radiation channels.

The inside of the reactor comprises: a central aluminium cylinder with a diameter of 3.8 cm, placed at the centre of the ring containing the combustible elements, a pneumatic "Rabbit" system, placed on the outermost ring that enables the introduction of the sample to be subject to radiation and its extraction from the reactor, a rotating disk, containing the housings for the cylindrical elements to be inserted inside them, positioned inside a circular well with internal walls of graphite, which performs the function of radial reflector, and, finally, a thermal channel, placed in the tank outside the graphite reflector.

At the start of the step for synthesising the active carriers 1, the inactive carriers 7 are preferably dosed using a dispenser for test tubes and weighed inside a polyethylene or quartz container using an analytical scale. The choice of polyethylene or quartz enables easier handling and transport.

The inactive carriers 7 in the polyethylene container, once weighed, are placed inside another container, designed for insertion in the thermal neutron reactor. The container containing the inactive carriers 7 is placed inside a housing of the rotating sample-carrier, preferably positioned so as to enable radiation with a neutron flow of 1x10¹² n cm⁻² s⁻¹.

In the variants of the process comprising holmium oxide, with the radiation the ¹⁶⁶Ho isotopes contained in the holmium oxide are converted, at least in part, into the ¹⁶⁶Ho isotopes. The neutron radiative capture reaction is, thus, ¹⁶⁵Ho (n,y) ¹⁶⁶Ho. The holmium isotope ¹⁶⁶Ho has a relative abundance of 100% and a collision section of 64 barns relating to the production of the ¹⁶⁶Ho isotope. The quantity of ¹⁶⁶Ho produced depends on the neutron radiation exposure time.

In particular, a similar reaction may also occur for the sodium typically contained in the glass nanospheres 3 like sodium oxide, the reaction: ²³Na (n,y) ²⁴Na. The content of sodium oxide in the glass nanospheres is typically between 12-14%.

If the neutron flow to which the inactive carriers 7 are subjected is higher than 1x10¹² n cm⁻² s⁻¹, the radiation time must, preferably, be reduced. Moreover, a high neutron flow tends to broaden the range of usable radionuclides.

At the end of the radionuclide generation step, the powder 2 is converted into activated powder 6 and, as a result, the coating 5 into the layer 9. Therefore, the inactive carriers 7 are converted into active carriers 1.

The process, finally, comprises a subsequent step of analysing the level of activity of the active carriers 1.

The analysis step is preferably carried out using gamma spectroscopy. In particular, in the case of activated powder 6 comprising holmium oxide, the level of activity of the ¹⁶⁶Ho isotopes of the active carriers 1 is measured.

The activity is measured after a waiting time after the radiation that must be measured for the purposes of calculating the activity or via the measurement of a portion. This procedure is useful for the active carrier 1 quality control steps, once the active carriers are produced and analysed.

The detector used for the measurement is preferably a high-purity germanium (HPGe) detector.

The instruments for gamma spectroscopy preferably comprise a multi-channel analyser, able to supplement the spectrum peak areas. The calibration of the energy and efficiency is preferably carried out using a standard source. The distance between the active carriers 1 and the detector is established, preferably based on the expected activity and the half-life of each type of radionuclide.

The activity is preferably measured by choosing the gamma peaks that have separate peak areas and a net area with an uncertainty below 30%.

At the end of the process, the active carriers 1 are obtained.

The active carriers 1 according to the invention achieve important advantages.

In fact, this new embodiment has the advantage of enabling the transport of the material containing radionuclides in constant quantities. This advantage is due to the best adhesion of the powder containing radionuclides to the surface of the nanospheres, as well as to the spherical form of the active carriers 1, compared known solutions.

Another advantage of this new solution is the greater ease in managing and dosing the active carriers 1 in medical applications. This advantage is due to the free flux properties of the glass nanospheres 3.

In addition, this solution has the advantage of not requiring additives to make the activated powder 6 adhere to the nanospheres.

Another important advantage is the reduction of the quantity of activated powder 6 used, since the best adhesion of the activated powder 6 to the surface of the nanospheres 3 reaches a higher level of activity of the active carriers 1 with the same amount of powder 2 used at the start of the process. This saving of material is due to the reduction of powder 2 detaching from the surface of the nanospheres 3.

The saving in material used also entails an economic advantage in the choice of this technical solution, since the materials typically chosen for generating radionuclides have high costs.

The better adhesion of the activated powder 6 to the surface of the nanospheres 3 also gives rise to the important advantage of reducing the dispersions of the powder in the environment and its inappropriate detachment in the patient.

These advantages linked to the increased adhesion of the activated powder 6 to the surface of the nanospheres 3 derive from the stage of grinding the powder 2 in order to avoid forming agglomerates, inside of which the particles are screened from the exposure to neutron beams during radiation.

Another advantage is linked to the use of nanospheres comprising silicon with an impurity level below 0.1%. In fact, as documented in the table below, the applicant has previously demonstrated that the use of nanospheres made of common glass also entails the activation of the elements present as impurities in the glass. The activation of the impurities entails subsequent, unwanted decay processes in the patient and must, therefore, be avoided. The table below shows the activity values of the elements present in the carrier samples comprising nanospheres made of common glass:

| **Sample** | **Ho 09** | | **Ho 10** | | **Ho 11** | | **Ho 12** | | **Ho 13** | | **Ho 14** | |
|---|---|---|---|---|---|---|---|---|---|---|---|---|
| **mass (g)** | **0.105** | | **0.1079** | | **0.1057** | | **0.108** | | **0.1098** | | **0.106** | |
| **Isotope** | **AV** | **SD** | **AV** | **SD** | **AV** | **SD** | **AV** | **SD** | **AV** | **SD** | **AV** | **SD** |
| Na-24 | | | | | | | | | 5.85 | 0.19 | 5.40 | 0.19 |
| Sc-46 | 545.14 | 0.03 | 520.27 | 0.03 | 553.78 | 0.03 | 541.97 | 0.03 | 61.52 | 0.04 | 59.15 | 0.05 |
| Cr-51 | 154.15 | 0.06 | 127.92 | 0.07 | 153.24 | 0.07 | 143.01 | 0.07 | 26.83 | 0.25 | 21.19 | 0.28 |
| Fe-59 | 36.64 | 0.09 | 41.48 | 0.09 | 0.00 | 0.00 | 11.32 | 0.26 | 13.42 | 0.17 | 8.14 | 0.27 |
| Co-60 | 9.52 | 0.12 | 4.00 | 0.30 | 10.90 | 0.10 | 9.51 | 0.11 | | | 12.59 | 0.09 |
| Zn-65 | 12.00 | 0.26 | 15.95 | 0.17 | 22.90 | 0.12 | 24.84 | 0.11 | 20.58 | 0.12 | 22.62 | 0.11 |
| Zr-95 | 20.37 | 0.14 | 22.55 | 0.13 | 18.47 | 0.16 | | | | | | |
| Nb-96 | 18.30 | 0.09 | 18.13 | 0.09 | 21.13 | 0.08 | 21.67 | 0.07 | 7.15 | 0.16 | 8.28 | 0.14 |
| Ag-110m | 5.22 | 0.22 | 0.74 | 0.00 | 0.76 | 0.00 | 0.76 | | 19.22 | | 16.93 | |
| Sb-124 | 14.29 | 0.11 | 20.32 | 0.08 | 10.80 | 0.15 | 12.50 | 0.12 | 171.54 | 0.03 | 165.53 | 0.03 |
| Cs-134 | 52.90 | 0.04 | 53.88 | 0.05 | 53.60 | 0.04 | 50.75 | 0.05 | 1.18 | 0.00 | 1.16 | |
| Ba-131 | 9.29 | 0.25 | 14.52 | 0.19 | 15.40 | 0.16 | | | 14.58 | 0.16 | 19.17 | 0.13 |
| Ce-141 | 19.49 | 0.06 | 18.57 | 0.07 | 19.04 | 0.07 | 19.63 | 0.07 | 34.84 | 0.05 | 30.02 | 0.05 |
| Eu-152 | 3.10 | 0.09 | 158.24 | 0.09 | 178.49 | 0.04 | 149.27 | 0.04 | 36.98 | 0.08 | 31.95 | 0.09 |
| Gd-153 | 34.61 | 0.09 | 24.26 | 0.12 | 24.13 | 0.13 | 28.33 | 0.11 | | | | |
| Tb-160 | 25.61 | 0.23 | 20.27 | 0.27 | | | 28.01 | 0.21 | | | | |
| **Ho-166m** | **32.88** | **0.04** | **68.55** | **0.04** | **86.85** | **0.04** | **95.33** | **0.04** | **95.74** | **0.04** | **96.41** | **0.04** |
| Tm-170 | 109.70 | 0.11 | 71.69 | 0.18 | 73.30 | 0.17 | 83.25 | 0.15 | 60.57 | 0.17 | 95.38 | 0.12 |
| Yb-169 | 11.04 | 0.25 | 13.74 | 0.20 | 13.89 | 0.20 | 11.03 | 0.28 | 9.25 | 0.29 | 9.71 | 0.21 |
| Hf-181 | 59.76 | 0.04 | 57.25 | 0.04 | 59.19 | 0.04 | 60.37 | 0.04 | 11.07 | 0.12 | 10.92 | 0.12 |
| Ta-182 | | | 42.67 | 0.12 | 46.10 | 0.11 | 38.82 | 0.13 | 13.45 | 0.27 | | |
| Pa-233 | 42.27 | 0.07 | 41.11 | 0.07 | 41.47 | 0.07 | 44.37 | 0.05 | 7.62 | 0.24 | 7.18 | 0.26 |

| | | | | | | | | | | | | |
|---|---|---|---|---|---|---|---|---|---|---|---|---|
| AV = Average value (Bq/g) SD = Standard deviation | | | | | | | | | | | | |

## Claims

1. A process for making active carriers (1)
said active carriers (1) comprising:
- nanospheres (3) of inorganic material,
- an organofunctional material (4), comprising at least epoxy-silane and preferably amino-silane,- an activated powder (6) made from material consisting at least in part of radionuclides,
- a layer (9) comprising said organofunctional material (4) and said activated powder (6) deposited on the surface of said nanospheres (3),
said process comprising
- a preparing step of said nanospheres (3) in inorganic material,
- a crushing step of a matrix from which a powder (2) is obtained and subsequent grinding,
- a preparation step of said organofunctional material (4),
and being **characterised by** comprising
- an initial step of synthesis of inactive carriers (7),
- said inactive carriers (7) comprising:
- said nanospheres (3),
- said powder (2),
- a coating (5) comprising said organofunctional material (4) and said powder (2) deposited on said surface of said nanospheres (3), and
- said first synthesis step comprising
- a sub-step of incorporation of said powder (2) into said organofunctional material (4),
- a sub-step of deposition of said organofunctional material (4) on said surface of said nanospheres (3) to achieve said coating (5),
- a second synthesis step of said active carriers (1), wherein said inactive carriers (7) are activated by means of:
- generation of radionuclides in said powder (2), by exposing said inactive carriers (7) to a neutron beam, and subsequent transformation of said powder (2) into activated powder (6), consequently of said coating (5) into said layer (9) and of said inactive carriers (7) into said active carriers (1),
- a subsequent step of analysing the level of activity of said active carriers (1).

2. The process according to claim 1, wherein said organofunctional material (4) comprises dipodal polysiloxane.

3. The process according to any one of the preceding claims, wherein said incorporation sub-step comprises preparing a dispersion (24) of said powder (2) in said organofunctional material (4).

4. The process according to any preceding claim, wherein in said deposition sub-step said nanospheres (3) are immersed in said dispersion (24) and said dispersion (24) adheres to said surface of said nanospheres (3) to make said coating (5).

5. The process according to any one of the preceding claims, wherein said nanospheres (3) comprise pure silica or glass without traces of CO₂ and sodium.

6. The process according to any one of the preceding claims, wherein said powder (2) comprises a metal belonging to the lanthanide series.

7. The process according to the preceding claim, wherein said powder (2) comprises holmium oxide.

8. The process according to any one of the preceding claims, wherein in said grinding step said powder (2) is dispersed in a dispersing agent (8) comprising isopropanol or silane gas.

9. Active carriers (1), made by the process according to any one of the preceding claims.

## Patentansprüche

1. Verfahren zur Herstellung aktiver Träger (1),
wobei die aktiven Träger (1) Folgendes umfassen:
- Nanosphären (3) aus anorganischem Material,
- ein organofunktionelles Material (4), umfassend zumindest Epoxysilan und vorzugsweise Aminosilan, - ein aktiviertes Pulver (6), das aus einem Material hergestellt ist, das zumindest teilweise aus Radionukliden besteht,
- eine Schicht (9), umfassend das organofunktionelle Material (4) und das aktivierte Pulver (6), die auf der Oberfläche der Nanosphären (3) abgeschieden ist,
wobei das Verfahren umfasst
- einen Schritt des Herstellens der Nanosphären (3) aus anorganischem Material,
- einen Zerkleinerungsschritt einer Matrix, aus der ein Pulver (2) erhalten wird, und ein anschließendes Mahlen,
- einen Schritt des Herstellens des organofunktionellen Materials (4),
und **dadurch gekennzeichnet ist, dass** es umfasst
- einen anfänglichen Syntheseschritt inaktiver Träger (7),
- wobei die inaktiven Träger (7) umfassen:
- die Nanosphären (3),
- das Pulver (2),
- eine Beschichtung (5), umfassend das organofunktionelle Material (4) und das Pulver (2), die auf der Oberfläche der Nanosphären (3) abgeschieden ist, und
- wobei der erste Syntheseschritt umfasst
- einen Teilschritt des Einbringens des Pulvers (2) in das organofunktionelle Material (4),
- einen Teilschritt des Abscheidens des organofunktionellen Materials (4) auf der Oberfläche der Nanosphären (3), um die Beschichtung (5) zu erhalten,
- einen zweiten Syntheseschritt der aktiven Träger (1), wobei die inaktiven Träger (7) aktiviert werden mittels:
- Erzeugung von Radionukliden in dem Pulver (2), indem die inaktiven Träger (7) einem Neutronenstrahl ausgesetzt werden, und anschließender Umwandlung des Pulvers (2) in aktiviertes Pulver (6), folglich der Beschichtung (5) in die Schicht (9) und der inaktiven Träger (7) in die aktiven Träger (1),
- einen anschließenden Schritt des Analysierens des Aktivitätsniveaus der aktiven Träger (1).

2. Verfahren nach Anspruch 1, wobei das organofunktionelle Material (4) dipodales Polysiloxan umfasst.

3. Verfahren nach einem der vorhergehenden Ansprüche, wobei der Teilschritt des Einbringens das Herstellen einer Dispersion (24) des Pulvers (2) in dem organofunktionellen Material (4) umfasst.

4. Verfahren nach einem der vorhergehenden Ansprüche, wobei in dem Teilschritt des Abscheidens die Nanosphären (3) in die Dispersion (24) eingetaucht werden und die Dispersion (24) an der Oberfläche der Nanosphären (3) haftet, um die Beschichtung (5) herzustellen.

5. Verfahren nach einem der vorhergehenden Ansprüche, wobei die Nanosphären (3) reines Siliciumdioxid oder Glas ohne Spuren von CO₂ und Natrium umfassen.

6. Verfahren nach einem der vorhergehenden Ansprüche, wobei das Pulver (2) ein Metall umfasst, das zur Reihe der Lanthanoide gehört.

7. Verfahren nach dem vorhergehenden Anspruch, wobei das Pulver (2) Holmiumoxid umfasst.

8. Verfahren nach einem der vorhergehenden Ansprüche, wobei in dem Mahlschritt das Pulver (2) in einem Dispergiermittel (8) dispergiert wird, das Isopropanol oder Silangas umfasst.

9. Aktive Träger (1), hergestellt durch das Verfahren nach einem der vorhergehenden Ansprüche.

## Revendications

1. Un procédé de fabrication de supports actifs (1)
lesdits supports actifs (1) comprenant :
- des nanosphères (3) en matériau inorganique,
- un matériau organofonctionnel (4), comprenant au moins de l'époxy-silane et
- de préférence de l'amino-silane,- une poudre activée (6) fabriquée à partir d'un matériau constitué au moins en partie de radionucléides,
- une couche (9) comprenant ledit matériau organofonctionnel (4) et ladite poudre activée (6) déposés sur la surface desdites nanosphères (3),
ledit procédé comprenant
- une étape de préparation desdites nanosphères (3) en matériau inorganique,
- une étape de concassage d'une matrice à partir de laquelle une poudre (2) est obtenue et un broyage subséquent,
- une étape de préparation dudit matériau organofonctionnel (4),
et étant **caractérisé en ce qu'**il comprend
- une étape initiale de synthèse de supports inactifs (7),
- lesdits supports inactifs (7) comprenant :
- lesdites nanosphères (3),
- ladite poudre (2),
- un revêtement (5) comprenant ledit matériau organofonctionnel (4) et ladite poudre (2) déposés sur ladite surface desdites nanosphères (3), et
- ladite première étape de synthèse comprenant
- une sous-étape d'incorporation de ladite poudre (2) dans ledit matériau organofonctionnel (4),
- une sous-étape de dépôt dudit matériau organofonctionnel (4) sur ladite surface desdites nanosphères (3) afin d'obtenir ledit revêtement (5),
- une deuxième étape de synthèse desdits supports actifs (1), dans laquelle lesdits supports inactifs (7) sont activés au moyen de :
- génération de radionucléides dans ladite poudre (2), par exposition desdits supports inactifs (7) à un faisceau de neutrons, et transformation subséquente de ladite poudre (2) en poudre activée (6), par conséquent dudit revêtement (5) en ladite couche (9) et desdits supports inactifs (7) en lesdits supports actifs (1),
- une étape subséquente d'analyse du niveau d'activité desdits supports actifs (1).

2. Le procédé selon la revendication 1, dans lequel ledit matériau organofonctionnel (4) comprend un polysiloxane dipodal.

3. Le procédé selon l'une quelconque des revendications précédentes, dans lequel ladite sous-étape d'incorporation comprend la préparation d'une dispersion (24) de ladite poudre (2) dans ledit matériau organofonctionnel (4).

4. Le procédé selon l'une quelconque des revendications précédentes, dans lequel, lors de ladite sous-étape de dépôt, lesdites nanosphères (3) sont immergées dans ladite dispersion (24) et ladite dispersion (24) adhère à ladite surface desdites nanosphères (3) pour réaliser ledit revêtement (5).

5. Le procédé selon l'une quelconque des revendications précédentes, dans lequel lesdites nanosphères (3) comprennent de la silice pure ou du verre sans traces de CO₂ et de sodium.

6. Le procédé selon l'une quelconque des revendications précédentes, dans lequel ladite poudre (2) comprend un métal appartenant à la série des lanthanides.

7. Le procédé selon la revendication précédente, dans lequel ladite poudre (2) comprend de l'oxyde d'holmium.

8. Le procédé selon l'une quelconque des revendications précédentes, dans lequel, lors de ladite étape de broyage, ladite poudre (2) est dispersée dans un agent dispersant (8) comprenant de l'isopropanol ou du gaz de silane.

9. Supports actifs (1), fabriqués par le procédé selon l'une quelconque des revendications précédentes.
